# EUROPEAN PATENT APPLICATION

(11) **EP 2 578 599 A1**
(43) Date of publication of application: **10.04.2013**
(21) Application number: 11184392.6
(22) Date of filing: 07.10.2011
(51) Int. Cl.: C07K 14/605

(54) **Cyclic analogs of GLP-1 and GLP-1 related peptides**

(71) Applicant: LanthioPep B.V., 9747 AG Groningen (NL)
(72) Inventor: Kuipers, Anneke, 8432 PL Haule (NL); de Vries, Louwe, 9951 NG Winsum (NL); Bosma, Tjibbe, 8408 HP Lippenhuizen (NL); Rink, Rick, 9717 AG Groningen (NL); Moll, Gert Nikolaas, 9725 LJ Groningen (NL)
(74) Representative: Jansen, Cornelis Marinus

(57) **Abstract**

The invention relates to cyclic peptide analogs of GLP-1 or of a GLP-1-related peptide, comprising at least one enzymatically formed intramolecular linkage between the side chains of two amino acids. The invention also relates to methods for the preparation of such cyclic peptide analogs and to the use thereof in therapy, for example metabolic diseases, neurological or neurodegenerative diseases and cardiovascular diseases.

## Description

The invention relates to cyclic peptide analogs and methods for the preparation thereof. In particular, the invention relates to pharmaceutically useful cyclic analogs of GLP-1 and of GLP-1 related peptides, such as glucagon, exendin4, VIP and GIP. The invention further relates to the use of such analogs as medicaments and to pharmaceutical compositions containing them.

Glucagon-like peptide-1 (GLP-1) is derived from proglucagon, which is produced in α-cells of the pancreas and in intestinal L-cells (for review see Mayo KE. et al. Pharmacol Rev 55(1): 167-194). In the intestinal L-cells GLP-1 is produced from proglucagon and secreted. GLP-1 belongs to the incretins, hormones that are produced by the gastrointestinal tract and that cause an increase in the amount of insulin released from the β-cells of the islets of Langerhans when glucose levels are normal or elevated. The biologically active forms of GLP-1 are GLP-1-(7-37) and GLP-1-(7-36)NH_{2,} which are both referred to as "GLP-1". The receptor for GLP-1 is the glucagon-like peptide 1 receptor (GLP1R), which is a member of the glucagon receptor family of G protein-coupled receptors. GLP1R is expressed in pancreatic beta cells.

GLP-1 exerts various biological activities, among which glucose-dependent stimulation of insulin secretion, suppression of glucagon secretion, inhibition of pancreatic β-cell apoptosis and stimulation of the proliferation and differentiation of insulin-secreting β-cells. GLP-1 thus is a potent antihyperglycemic hormone. GLP-1 further inhibits gastric emptying and motility, contributing to satiation. For a review on GLP-1 and

As a result of its biological activities, GLP-1 has received a lot of attention as a potential therapeutic agent for treatment of diabetes mellitus. Cardiovascular disease is the leading cause of death in patients with type 2 diabetes mellitus. GLP-1 has been demonstrated also to have direct cardioprotective effects, independent of glucose and may therefore exert beneficial cardiovascular effects independent of its glucose-lowering actions. Furthermore, emerging evidence suggests that neuronal GLP-1 receptor stimulation plays a significant role in regulating neuronal plasticity and cell survival. GLP-1 and exendin-4, a GLP-1-related peptide that likewise binds to the GLP-1 receptor, were shown to reduce endogenous levels of amyloid-beta peptide in mouse brain and to reduce levels of beta-amyloid precursor protein in neurons. Therefore, GLP-1 also has therapeutic potential in specific neurological and neurodegenerative conditions.

An important drawback of the therapeutic use of native GLP-1 is its plasma half-life of less than 2 minutes, due to rapid degradation by the enzyme dipeptidyl peptidase-4 (DPP-IV). DPP-IV cleaves GLP-1 between Ala⁸ and Gln⁹. Another enzyme responsible for cleavage of GLP-1 is neutral endopeptidase (NEP) 24.11, which cleaves at multiple sites between amino acid positions 15 and 32. As a result, the use of GLP-1 in clinical therapy is severely hampered. Therefore, synthetic analogues of GLP-1 (such as exenatide and liraglutide) that are less sensitive to degradation by DPP-IV have been developed as therapeutic antidiabetic strategy. In addition, pharmacological inhibitors of DPP-IV are used to increase endogenous levels of GLP-1. Examples of such inhibitors are sitagliptin and vildagliptin.

Other approaches to increase stability of GLP-1 include replacement of the alanine at position 8 by a non-cleavable residue, e.g. glycine, D-alanine, alpha-aminoisobutyric acid (Aib), serine or threonine, to induce resistance to DPPIV (Xiao Q. et al. Biochemistry, 2001, 40(9):2860-9; Deacon CF. et al. Diabetologia, 1998, 41(3):271-8), or derivatisation of GLP-1 with fatty acids (Knudsen LB. et al. J Med Chem. 2000 43(9):1664-9). However, the increase in plasma half-life of such GLP-1 analogs is limited; substitution of Ala⁸ by glycine or Aib only increased plasma half-life to 3-4 minutes.

Further described in the art are cyclised GLP-1 analogs which have increased plasma stability (Miranda et al. (J. Med. Chem. 2008, 51: 2758-2765), W02007/124461, Murage et al. (J. Med. Chem. 2010, 53: 6412-6420), WO 2010/118034). Miranda *et al*. disclose the design of GLP-1(1-37)-NH₂ analogs containing lactam bridges between the side chains of amino acids glutamic acid and lysine, which are separated by three, four or six other amino acids, and which are located between positions 18 and 30. Some of the cyclised analogs wherein the lactam bridge spans three amino acids show increased plasma stability as compared to GLP-1. *In vivo* functional activity was only tested using PEGylated cyclic GLP-1 analogs. GLP-1 analogs containing one, two or three lactam bridges are also described by Murage *et al*., reporting increases in DPPIV and NEP 24.11 resistance depending on the number, location and orientation of the lactam bridges.

A major disadvantage of the known cyclic GLP-1 analogs containing lactam bridges is the need for chemical synthesis of the analogs, e.g. solution-phase synthesis or solid-phase synthesis using for instance *N*^{α}-Fmoc/*tert*-butyl chemistry. This technique involves the synthesis of a polypeptide one amino acid at a time and may require the isolation of all peptide intermediates and is therefore very laborious. In particular, the synthesis of large amounts of these lactam containing GLP-1 analogs for therapeutic purposes is problematic. The limited capacity of solid supports has restricted the use of solid phase methodology. Other disadvantages of solid-phase synthesis include the occurrence of incomplete reactions, aggregation of peptide sequences during synthesis, and lack of stereo-, regio- and/or chemo-specificity.

It is therefore an aim of the present invention to provide a new class of cyclic analogs of GLP-1 and GLP-1 related peptides that can be obtained in a way that is less laborious and economically more attractive as compared to known analogs, and which allows for large scale production. Preferably, the cyclic analogs have enhanced resistance to proteolytic degradation as compared to their linear counterpart.

The present invention provides the insight that the introduction of an enzymatically formed intramolecular linkage between the side chains of two amino acids of GLP-1 and GLP-1 related peptides results in analogs having an enhanced biological activity. The increased proteolytic resistance is particularly advantageous for their therapeutic use and allows for a lower dose and/or a lower frequency of administration, and may allow effective oral administration. Furthermore, enzymatic formation of intramolecular linkages allows for production of cyclic analogs in host cells thereby obviating the need for chemical synthesis.

Accordingly, the invention provides a cyclic peptide analog of GLP-1 or of a GLP-1-related peptide, comprising at least one enzymatically formed intramolecular linkage between the side chains of two amino acids. Cyclic peptide analogs of a GLP-1 (related) peptide, wherein the linkage is formed enzymatically have not been previously disclosed. Enzymatic formation of the intramolecular linkages allows for the preparation of the analogs in an *in vitro* system without the need for conventional chemical synthesis methods as described above. Preparation of the analogs according to the invention is thus not associated with the above mentioned disadvantages of such conventional synthesis methods.

The present inventors found that enzymatically formed intramolecular linkages are particularly suitable to stabilize cyclic peptide analogs. GLP-1 and GLP-1-related peptide contain a C-terminal alpha-helix structure which is needed for receptor binding. GLP-1, for instance, has an alpha-helix from Thr¹³ to Val³³ when bound to the extracellular domain of the GLP-1 receptor. An intramolecular linkage enzymatically formed between the side chains of two amino acid side is thought to stabilize the alpha-helix structure of the analog and, by that, the three dimensional structure of the C-terminus. Importantly, it was also found that the introduction of enzymatically formed intramolecular linkages yields highly active cyclic peptide analogs. As for example shown in Example 2, cyclic GLP-1 analogs are provided having a lysinoalanine at Xaa²⁶ and Xaa³⁰, or Xaa³⁰ and Xaa³⁴, or a lanthionine at Xaa¹⁸ and Xaa²¹, that have a high activity. GLP-1 [lysinoalanine Xaa³⁰-Xaa³⁴] is even more active than wild type GLP-1. In view of this high activity in addition to their enhanced resistance to proteolytic cleavage, GLP-1 analogs are obtained with enhanced biological activity as compared to wild type, linear GLP-1.

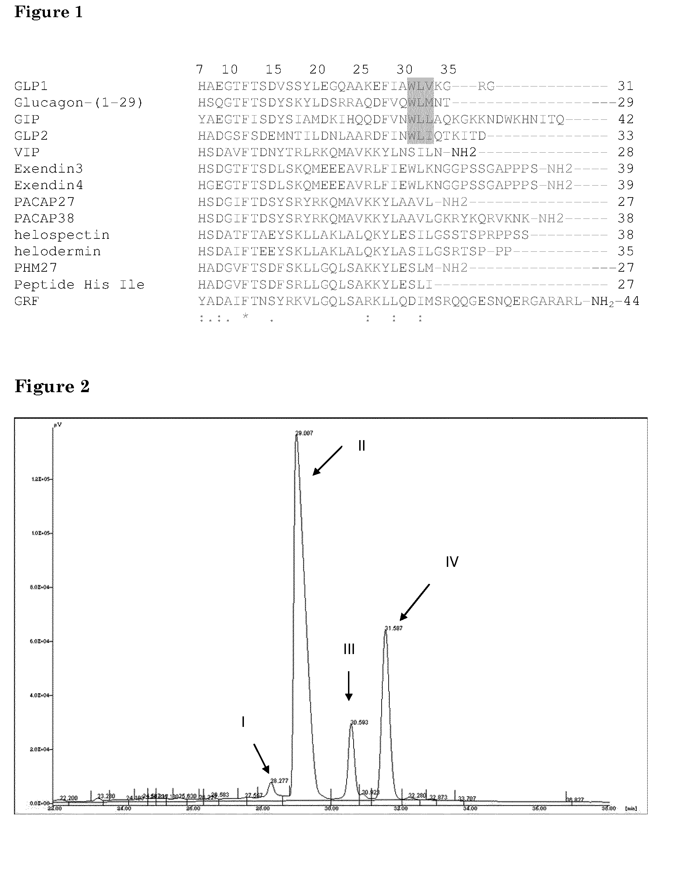

Enzymatically formed intramolecular linkages are formed between the side chain of an enzymatically dehydrated serine or threonine and the side chain of a cysteine or a lysine. Prior to the present invention, it was not known that at a pre-determined, specific location within the GLP-1 (related) peptide analog intramolecular linkages can be formed enzymatically. As can be seen in figure 1, GLP-1 (related) peptides contain serines and threonines at several positions, some of which are essential for biological activity, such as Thr¹³ in GLP-1 and many of the GLP-1 related peptides. It would therefore be expected that, in addition to dehydration of serine or threonine residues intended to be participate in the intramolecular linkage, other serines and threonines will also be dehydrated enzymatically. Such dehydration of (essential) serine and/or threonine residues is very likely to result in analogs that have no biological activity. Surprisingly, it was found that most serine and threonine residues present in a GLP-1 (related) peptide are not or only slightly sensitive to enzymatic modification. Only Ser¹⁷, which is not essential for biological activity, is sensitive to enzymatic modification and may interfere with intramolecular linkage formation, depending on the location of the linkage within the analog. Hence, enzymatic formation of intramolecular linkages can unexpectedly be achieved without further modification, e.g. replacement of serine or threonine residues that will not be part of the intramolecular linkage, or with the replacement of only Ser¹⁷, depending on the location of the linkage within the analog.

A "cyclic peptide analog of GLP-1 or of a GLP-1-related peptide" refers to any analog of GLP-1 or a GLP-1 related peptide being capable of selective receptor binding and having biological activity, and which comprises at least one intramolecular linkage. Selective receptor binding refers to the selective binding of a GLP-1 analog to GLP1R, and likewise, where the analog is a GLP-1 related peptide analog to the receptor of said GLP-1 related peptide, for example the glucagon receptor where the analog is a cyclic glucagon analog and VPAC₁ or VPAC₂ where the analog is a cyclic VIP analog.

A cyclic peptide analog has at least one enzymatically formed intramolecular linkage between the side chains of two amino acids. It may have more than one enzymatically formed intramolecular linkage. For instance, in one embodiment, a cyclic peptide analog has two enzymatically formed intramolecular linkages between the side chains of two amino acids. In another embodiment, a cyclic peptide analog has three enzymatically formed intramolecular linkages between the side chains of two amino acids. Cyclic peptide analogs of GLP-1 or of GLP-1-related peptides comprising at least one enzymatically formed intramolecular linkage between the side chains of two amino acids are herein also referred to as "cyclic peptide analogs according to the invention", "cyclic peptide analogs" or "analogs".

One or more amino acid residues of a cyclic peptide analog according to the invention may have been altered as compared to a GLP-1 (related) peptide at a position which forms part of the intramolecular linkage. Such amino acid residues can be substituted to allow for the formation of the intramolecular linkage. Amino acids which are not part of an intramolecular linkage can be identical to the amino acid at the corresponding position in the linear counterpart a GLP-1 (related) peptide. However, conservative amino acid substitution of one or more amino acid residues is allowed in a cyclic peptide analog. "Conservative amino acid substitution" as used herein refers to the substitution of one amino acid for another at a given location in a cyclic peptide analog, where the substitution can be made without substantial loss of biological activity. Amino acid substitution can be made based on relative similarity of the side-chains of amino acids, for example, based on their size, charge, hydrophobicity or hydrophilicity, or based on the division of amino acids into non-polar (Ala, Val, Leu, Ile, Phe, Trp, Pro, Met), acidic (Asp, Glu), basic (Lys, Arg, His) and neutral (Gly, Ser, Thr, Cys, Asn, Gin, Tyr) classes. Conservative amino acid substitution can also include the substitution of an L-amino acid by the corresponding D-amino acid. Whether an amino acid substitution can be made without substantial loss of biological activity can be determined on the basis of activity assays well known in the art, for instance using any *in vitro* assay system as described herein elsewhere.

If the cyclic peptide analog is an analog of GLP-1, amino acid residues at positions 7, 10, 12, 13, 15, 28 and/or 29 are preferably not substituted by other amino acids, because this is known to result in loss or reduction of biological activity. A cyclic peptide analog preferably has at least 75% amino acid sequence identity with its linear counterpart GLP-1 or GLP-1 related peptide. More preferably, a cyclic peptide analog has at least 80%, more preferably at least 85%, 86%, 87%, 88%, 89% or 90% sequence identity with its linear counterpart GLP-1 or GLP-1 related peptide. "% sequence identity" is defined herein as the percentage of amino acid residues in a cyclic peptide analog that is identical with the amino acid residues in its linear counterpart GLP-1 (related) peptide after aligning the two sequences and introducing gaps, if necessary, to achieve the maximum percent identity. Methods and computer programs for sequence alignment are well known in the art. For instance, a computer program which may be used for purposes of determining sequence identity is "Align 2".

As used herein, "GLP-1" refers to both GLP-1-(7-37) and GLP-1-(7-36)NH2. Native GLP-1-(7-37) has the amino acid sequence His⁷-Ala⁸-Glu⁹-Gly¹⁰-Thr¹¹-Phe¹²-Thr¹³-Ser¹⁴-Asp¹⁵-Val¹⁶-Ser¹⁷-Ser¹⁸-Tyr¹⁹-Leu²⁰-Glu²¹-Gly²²-Gln²³-Ala²⁴-Ala²⁵-Lys²⁶-Glu²⁷-Phe²⁸-Ile²⁹-Ala³⁰- Trp³¹-Leu³²-Val³³-Lys³⁴-Gly³⁵-Arg³⁶-Gly³⁷. The numbering of amino acid residues in GLP-1, GLP-1 related peptides and cyclic peptide analogs according to the invention as used herein is based on the numbering of GLP-1 after cleavage from proglucagon, which is as depicted in figure 1. In a preferred embodiment, a cyclic peptide analog of GLP-1 (PDB accession no. 3IOL_B) is provided.

As used herein, GLP-1-related peptides are peptides which are structurally related to GLP-1. Figure 1 shows the amino acid sequence of GLP-1 and several peptides that are structurally related to GLP-1, and their mutual homology. GLP-1 (related) peptides share a DPP IV cleavage site between residue 8 and 9 of GLP-1. Position 7 to 12 or homologous positions are rather conserved. Position 7 is a histidine or a tyrosine, responsible for signal transduction. Position 8 is serine, alanine or glycine. Position 9 is mostly aspartate or glutamate. Position 10 is glycine or alanine; position 12 is phenylalanine, position 13 is mostly threonine. The C-terminal part is alpha helical)) In one embodiment, a GLP-1-related peptide is selected from the group consisting of GLP-2 (PDB accession no. 2L63_A), glucagon (PRF accession no. 570104A), GIP (PDB accession no. 2B4N_A), exendin-4 (GenBank accession no. AAB22006), exendin-3 (Swiss-Prot accession no. P20394), VIP (PRF accession no. 0601216A), PACAP27, PACAP38 (GenBank accession no. AAB20402), helospectin (Swiss-Prot accession no. P04203), helodermin (Swiss-Prot accession no. P04204), PHM27 (PRF accession no. 1010243A), peptide His Ile (PRF accession no. 1011215A) and GRF (GenBank accession no. CAA24956).

In one embodiment a cyclic peptide analog of glucagon is provided, the analog comprising at least one enzymatically formed intramolecular linkage between the side chains of two amino acids. Glucagon is a peptide hormone related to GLP-1. Glucagon is also derived from proglucagon. However, as opposed to GLP-1, glucagon is produced in the α-cells of the islets of Langerhans and secreted by the pancreas. Glucagon raises blood glucose levels. Its effect is opposite that of insulin, which lowers blood glucose levels. The pancreas releases glucagon when blood glucose levels become too low. Glucagon stimulates the liver to convert glycogen into glucose, which is released into the bloodstream. High blood glucose levels stimulate the release of insulin. Insulin allows glucose to be taken up and used by insulin-dependent tissues. An exemplary cyclic glucagon analog of the invention comprises at least one enzymatically formed intramolecular linkage between the side chains of Xaa³⁰ and Xaa³⁴.

Glucagon-like peptide-2 (GLP-2) is, like GLP-1, produced from proglucagon by the intestinal endocrine L cell, and also by neurons in the central nervous system. In another embodiment, therefore, a cyclic GLP-2 analog is provided, which analog comprises at least one enzymatically formed intramolecular linkage between the side chains of two amino acids. GLP-2 is a 33 amino acid peptide. Intestinal GLP-2 is cosecreted with GLP-1 upon nutrient ingestion. GLP-2 has a number of effects including intestinal growth, enhancement of intestinal function, reduction in bone breakdown and neuroprotection. An exemplary cyclic GLP-2 analog of the invention comprises at least one enzymatically formed intramolecular linkage between the side chains of Xaa¹⁸ and Xaa²², and/or Xaa³⁰ and Xaa³⁴_{.}

In yet another embodiment a cyclic peptide analog of exendin-3 or exendin-4 is provided, the analog comprising at least one enzymatically formed intramolecular linkage between the side chains of two amino acids. Exenatide, mentioned above as a synthetic analogue of GLP-1, is a synthetic version of exendin-4, a peptide present in saliva of Gila monster (*Heloderma suspectum*). Another peptide related to GLP-1 and Exendin-4 is Exendin-3, which is present in saliva from the beaded lizard (*Heloderma horridum*). Exendin-3 and Exendin-4 are also peptide hormones and differ in only two amino acids. An exemplary cyclic Exendin analog of the invention comprises at least one enzymatically formed intramolecular linkage between the side chains of Xaa¹⁷ and Xaa²⁰, and/or Xaa³⁰ and Xaa33.

Vasoactive intestinal peptide (VIP), like GLP-1, belongs to the secretin family. Also provided is therefore a cyclic VIP analog comprising at least one enzymatically formed intramolecular linkage between the side chains of two amino acids. VIP is a peptide hormone containing 28 amino acid residues and is produced in many areas of the human body including the intestine, pancreas and the hypothalamus. In humans, the vasoactive intestinal peptide is encoded by the *VIP* gene. Like, GLP-1, VIP has a half-life (T^{1/2}) in the blood of about two minutes. An exemplary cyclic VIP analog of the invention comprises at least one enzymatically formed intramolecular linkage between the side chains of Xaa²⁰ and Xaa²⁴, and/or Xaa³¹ and Xaa³⁴.

In yet another embodiment a cyclic GIP analog of is provided, which analog comprises at least one enzymatically formed intramolecular linkage between the side chains of two amino acids. Gastric inhibitory polypeptide (GIP) also belongs to the incretins. Biologically active GIP is a 42-amino acid peptide derived from a proprotein encoded by the GIP gene It is synthesized by K cells, which are present in the mucosa of the duodenum and the jejunum of the gastrointestinal tract. Like, glucagon, GIP induces insulin secretion. An exemplary cyclic GIP analog of the invention comprises at least one enzymatically formed intramolecular linkage between the side chains of Xaa¹⁹ and Xaa²³, and/or Xaa³⁰ and Xaa³⁴_{.}

In yet another embodiment a cyclic peptide analog of PACAP27 or PACAP38 is provided, the analog comprising at least one enzymatically formed intramolecular linkage between the side chains of two amino acids. Pituitary adenylate cyclase-activating polypeptide (PACAP) exists in two bioactive forms, one of 38 amino acids (PACAP38) and a shorter form consisting of the N-terminal 27 amino acids of PACAP38 (PACAP27). It was discovered as an ovine hypothalamic neuropeptide. It is abundant in both the central and peripheral nervous systems and exerts a variety of effects, including being parasympathetic and sensory neurotransmitter, stimulation of insulin secretion from islets in a glucose-dependent manner and being an insulinotropic factor. An exemplary cyclic PACAP38 analog of the invention comprises at least one enzymatically formed intramolecular linkage between the side chains of Xaa²⁴ and Xaa²⁷, and/or Xaa³¹ and Xaa³⁵.

Helospectin I, helospectin II and helodermin are further short peptides related to GLP-1. Helospectin I, helospectin II and helodermin are originally isolated from isolated from the salivary gland venom of Heloderma suspectum. Helospectin I and II co-localizes with VIP in nerve fibers surrounding vascular smooth muscle. Provided is therefore also a cyclic peptide analog of helospectin I, helospectin II and helodermin comprising at least one enzymatically formed intramolecular linkage between the side chains of two amino acids. An exemplary cyclic Helospectin/helodermin analog of the invention comprises at least one enzymatically formed intramolecular linkage between the side chains of Xaa²⁰ and Xaa²³, or Xaa²⁰ and Xaa²⁴, and/or Xaa¹⁸ and Xaa²².

In yet another embodiment a cyclic PHM-27 analog is provided, the analog comprising at least one enzymatically formed intramolecular linkage between the side chains of two amino acids. PHM-27 is a 27 amino acid peptide. It is secreted in the intestine, but is found in many organs, the nervous system, and in the majority of peripheral tissues. It has a wide range of biological actions, affecting the cardiovascular, gastrointestinal, respiratory, and central nervous systems. An exemplary cyclic PHM analog of the invention comprises at least one enzymatically formed intramolecular linkage between the side chains of Xaa¹⁷ and Xaa²¹, or Xaa¹⁹ and Xaa²², and/or Xaa²⁷ and Xaa²⁷.

Further provided is a cyclic peptide His Ile analog comprising at least one enzymatically formed intramolecular linkage between the side chains of two amino acids. Peptide His Ile is a peptide hormone which shows homology to VIP. It plays a role in the regulation of prolactin in humans. An exemplary cyclic His Ile analog of the invention comprises at least one enzymatically formed intramolecular linkage between the side chains of Xaa¹⁷ and Xaa²¹, and/or Xaa²⁴ and Xaa²⁷.

A further peptide related to GLP-1 is growth-hormone-releasing factor (GRF or GJRF). GRF is also known as Growth-hormone-releasing hormone (GHRH). It is a 44-amino acid peptide hormone produced in the arcuate nucleus of the hypothalamus, and is a releasing hormone for growth hormone. In a further embodiment a cyclic GRF analog is provided, the analog comprising at least one enzymatically formed intramolecular linkage between the side chains of two amino acids. An exemplary cyclic GRF analog of the invention comprises at least one enzymatically formed intramolecular linkage between the side chains of Xaa¹⁸ and Xaa²², and/or Xaa²⁴ and Xaa²⁸.

The term "intramolecular linkage" as used herein refers to a linkage between the side chains of two amino acids within a single analog of GLP-1 or of a GLP-1-related peptide. An intramolecular linkage is herein also referred to as a "bridge" or "cyclic structure". The formation of such intramolecular linkage results in the analog adopting a cyclised structure. The size of the intramolecular linkage can vary. The two amino acids of a cyclic peptide analog forming the cyclic structure can be adjacent to each other. Preferably, however, these two amino acids are separated by 1 or more amino acids, such as by 1, 2, 3, 4, 5, 6, or 7 amino acids. In one embodiment, the two amino acids are separated by 2 amino acids. Thioether bridged peptide analogs in which the linkage spans 2 or 3 amino acids that are not part of the linkage have high biological activity. Therefore, the two amino acids forming a cyclic lanthionine or methyllanthionine structure are preferably separated by 3 amino acids. In another embodiment, the two amino acids are separated by 4 amino acids. Enzymatically formed intramolecular linkages bridging more than two amino acids are at present not known to occur naturally. Surprisingly, however, it was found that in particular cyclic peptide analogs in which an intramolecular lysinoalanine linkage spans 3 amino acids have high biological activity. Examples of analogs with high biological activity are those having a bridge between Xaa¹⁸ and Xaa²² and/or between Xaa³⁰ and Xaa³⁴. Therefore, the two amino acids forming the cyclic lysinoalanine structure are preferably separated by 3 amino acids. As used herein, "separated by, i.e., 3 amino acids" indicates that three amino acids are positioned between the two amino acids forming the intramolecular linkage in a cyclic peptide analog according to the invention. For instance, Xaa²² and Xaa²⁶ are separated by 3 amino acids.

"Enzymatically formed" as used herein indicates that at least one stage of the formation of an intramolecular linkage is induced by an enzyme. The first stage in an advantageous example of formation of an intramolecular linkage is the dehydration of at least one of the two amino acids of which the side chains will form the intramolecular linkage. A second, subsequent, stage is the interaction between the side chains of the two amino acids to form the cyclic structure. Thus, for example, both stages can be induced by one or more enzymes. As another example, dehydration of at least one of the two amino acids is induced by an enzyme, followed by spontaneous interaction between the side chains of the two amino acids after exposure of the analog to an increased pH, of which at least one is dehydrated.

In a preferred embodiment, a cyclic peptide analog according to the invention has enhanced biological activity as compared to the linear counterpart GLP-1 or GLP-1 related peptide. "Biological activity" as used herein refers to *in vitro* or *in vivo* biological activity of the cyclic peptide analog. Biological activity of a cyclic peptide analog can be enhanced as a result of a variety of mechanisms. For instance, the receptor affinity or receptor interaction of a cyclic peptide analog is increased as compared to its linear counterpart. Another possibility is that a cyclic peptide analog has enhanced biological activity because the analog has increased plasma stability and consequently has an enhanced bioavailability, as a result of enhanced resistance to proteolytic cleavage as compared to the linear counterpart. Preferably, the affinity of a cyclic peptide analog to its receptor of a cyclic peptide analog is at least comparable to the affinity of the linear counterpart. However, it is not necessary for a cyclic peptide analog having enhanced resistance to proteolytic cleavage to have an affinity as high as the linear counterpart. A reduced receptor interaction, but a strongly enhanced bioavailability may result in an enhanced biological activity. A loss of 90% of activity upon ring introduction can still yield a cyclic peptide analog of great interest if its stability is 1000 times higher than its linear counterpart. In one embodiment, a cyclic peptide analog is provided which as en enhanced in vivo efficacy.

A "linear counterpart GLP-1 or GLP-1 related peptide" refers to naive, unmodified GLP-1, or naive, unmodified GLP-1 related peptide, such as GLP-2, glucagon, GIP, exendin-4, exendin-3, VIP, PACAP27, PACAP38, helospectin, helodermin, PHM27, peptide His Ile or GRF, of which the cyclic peptide is an analog.

Biological activity of a cyclic peptide analog can be determined using methods known in the art, including radioligand binding studies, *in vitro* cell activation assays and *in vivo* experiments. See for example Koziarz et al., 1993 Gen. Pharmacol. 24, 705-713; Lemos et al 2005 J Cardiovasc Pharmacol 46, 274-279; Santos et al 2003 PNAS 100: 8258-8263; Godeny and Sayeski 2006 Am J Physiol Cell Physiol.;291(6):C1297-307; Sarr et al., Cardiovasc Res. 2006 Sep 1;71(4):794-802; ; Silva et al 2007 Peptides 28, 702-707.

An example of an *in vitro* cell activation assay which can be used to determine biological activity is described in Example 2, which involves a cyclic peptide analog and a competitor, such as the linear counterpart GLP-1 (related) peptide. Said biological activity measured is for instance cAMP production in a reporter cell line, which is for instance determined by ELISA. Assays that can be used to determine resistance to proteolytic cleavage include, but are not limited to, measuring *in vitro* plasma stability, and direct measurement of degradation of an analog and its linear counterpart by proteolytic enzymes, such as DPP-IV and/or NEP 24.11. This involves the incubation of the analog and its linear counterpart in human plasma or with proteolytic enzymes and subsequently determining the concentration of intact analog and linear counterpart by, for instance, HPLC.

As described above, bridges in cyclic peptide analogs are preferably formed between the side chains of amino acid residues which are separated by 2, 3 or 4 other amino acids. Furthermore, amino acids His⁷, Gly¹⁰, Phe¹², Thr¹³, Asp¹⁵, Phe²⁸ and Ile²⁹ of cyclic GLP-1 (related) peptides are preferably not substituted by other amino acids because of the risk of reducing or eliminating biological activity. Therefore, intramolecular linkages are preferably formed between the side chains of two amino acids other than His⁷, Gly¹⁰, Phe¹², Thr¹³, Asp¹⁵, Phe²⁸ and Ile²⁹. A cyclic peptide analog according to the invention preferably comprises at least one enzymatically formed intramolecular linkages between the side chains of the amino acids corresponding to positions Xaa⁸ and Xaa¹¹, and/or
Xaa¹¹ and Xaa¹⁴, and/or Xaa¹⁴ and Xaa¹⁷, and/or Xaa¹⁴ and Xaa¹⁸, and/or Xaa¹⁴ and Xaa¹⁹, and/or Xaa¹⁶ and Xaa¹⁹, and/or Xaa¹⁶ and Xaa²⁰, and/or Xaa¹⁶ and
Xaa²¹, and/or Xaa¹⁷ and Xaa²⁰, and/or Xaa¹⁷ and Xaa²¹, and/or Xaa¹⁷ and Xaa²², and/or Xaa¹⁸ and Xaa²¹, and/or Xaa¹⁸ and Xaa²², and/or Xaa¹⁸ and Xaa²³, and/or Xaa¹⁹ and Xaa²², and/or Xaa¹⁹ and Xaa²³, and/or Xaa¹⁹ and Xaa²⁴, and/or Xaa²⁰ and Xaa²³, and/or Xaa²⁰ and Xaa²⁴, and/or Xaa²⁰ and Xaa²⁵, and/or Xaa²¹ and Xaa²⁴, and/or Xaa²¹ and Xaa²⁵, and/or Xaa²¹ and Xaa²⁶, and/or Xaa²² and Xaa²⁵, and/or Xaa²² and Xaa²⁶, and/or Xaa²² and Xaa²⁷, and/or Xaa²³ and Xaa²⁶, and/or Xaa²³ and Xaa²⁷, and/or Xaa²⁴ and Xaa²⁷, and/or Xaa²⁵ and Xaa³⁰, and/or Xaa²⁶ and Xaa³⁰, and/or Xaa²⁶ and Xaa³¹, and/or Xaa²⁷ and Xaa³⁰, and/or Xaa²⁷ and Xaa³¹, and/or Xaa²⁷ and Xaa³², and/or Xaa³⁰ and Xaa³³, and/or Xaa³⁰ and Xaa³⁴, and/or Xaa³⁰ and Xaa³⁵, and/or Xaa³¹ and Xaa³⁴, and/or Xaa³¹ and Xaa³⁵, and/or Xaa³¹ and Xaa³⁶, and/or Xaa³² and Xaa³⁵, and/or Xaa³² and Xaa³⁶, and/or Xaa³² and Xaa³⁷, and/or Xaa³³ and Xaa³⁶, and/or Xaa³³ and Xaa³⁷, and/or Xaa³⁴ and Xaa³⁷.

Preferably, intramolecular linkages are formed between the side chains of the amino acids corresponding to positions Xaa¹⁸ and Xaa²¹, and/or Xaa¹⁸ and Xaa²², and/or Xaa³⁰ and Xaa³⁴, and/or Xaa³¹ and Xaa³⁴ as defined in figure 1. More preferably a cyclic peptide analog according to the invention comprises at least one intramolecular linkages which is formed between the side chains of the amino acids corresponding to positions Xaa³⁰ and Xaa³⁴, preferably a lysinoalanine. As demonstrated in Example 2, a cyclic GLP-1 analog with a lysinoalanine between positions Xaa³⁰ and Xaa³⁴ has a particularly high activity. Furthermore, activity of this cyclic GLP-1 analog is not antagonized by exendin-9, a GLP-1 receptor antagonist. Another preferred cyclic peptide analog according to the invention comprises at least one intramolecular linkages which is formed between the side chains of the amino acids corresponding to positions Xaa¹⁸ and Xaa²¹, preferably a lanthionine or a methyllanthionine. In Example 3 it is shown that induction of a thioether bridge between positions Xaa¹⁷ and Xaa²¹ results in an active cyclic GLP-1 analog. Although the *in vitro* activity is reduced as compared to the linear counterpart GLP-1, a strongly enhanced resistance to proteolytic cleavage may result in a GLP-1 analog having net enhanced biological activity.

In one embodiment, an enzymatically formed intramolecular linkage has the general formula I: 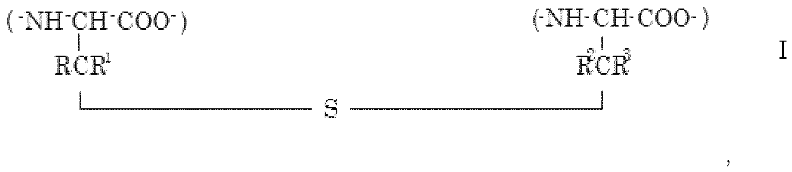 wherein R¹, R² and R³ are independently selected from -H, a lower (e.g. C₁-C₁₀) alkyl or aralkyl group, preferably wherein R¹, R² and R³ are independently selected from H and CH₃.

A structure according to formula I is herein also referred to as thioether cross-linked amino acids, a lanthionine in the case of Ala-S-Ala, or a methyllanthionine in the case of Abu-S-Ala or Ala-S-Abu, wherein Abu is a dehydrobutyrine.

Lanthionine and methyllanthionine are preferably formed between the side chains of two amino acids separated by two or three amino acids because of the relatively small size of the thioether bridge. Lanthionines and/or methyllanthionines are preferably formed between the side chains of amino acids Xaa¹⁸ and Xaa²¹, and/or Xaa¹⁸ and Xaa²², and/or Xaa³⁰ and Xaa³⁴, and/or Xaa³¹ and Xaa³⁴, of a cyclic peptide analog according to the invention. Preferably, said cyclic peptide analog is an analog of GLP-1.

In another embodiment an enzymatically formed intramolecular linkage has the general formula II or III: 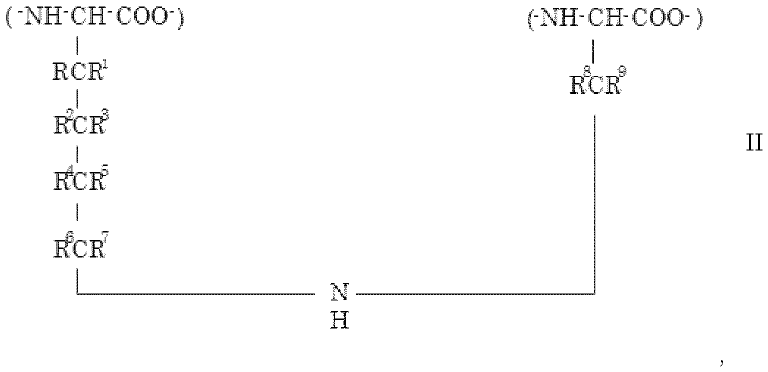 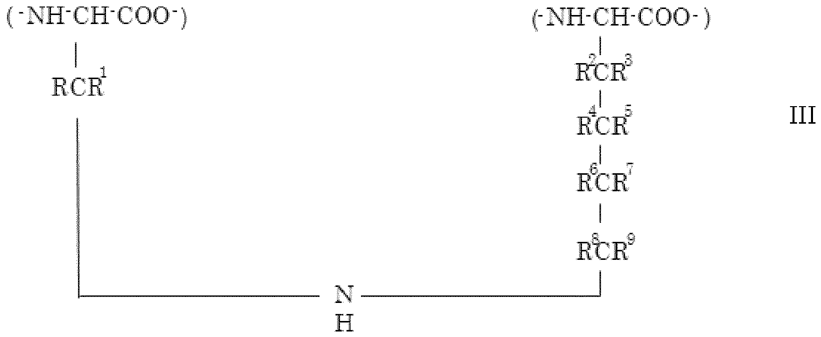 wherein R¹, R², R³, R⁴, R⁵, R⁶, R⁷, R⁸ and R⁹ are independently selected from -H, a lower (e.g. C₁-C₁₀) alkyl or aralkyl group, preferably wherein R¹, R², R³, R⁴, R⁵, R⁶, R⁷, R⁸ and R⁹ are independently selected from H and CH₃.

A structure according to formula II or III is herein also referred to as a lysinoalanine.

"Alkyl" as used herein is defined as a branched or straight chain consisting of 1-10 carbons atoms.

"Aralkyl" as used herein is defined as an aryl group having an alkyl group as defined above attached to the aryl group, wherein "aryl" is defined as a C6-C10 aromatic ring system.

A lysinoalanine is preferably formed between the side chains of two amino acids separated by two, three or four amino acids. As described herein before, enzymatically formed cyclic structures bridging more than two amino acids are at present not known to occur naturally. Surprisingly, it was found that in particular cyclic peptide analogs in which a bridge having the structure of formula II or of formula III spanning three amino acids have high biological activity. Examples of analogs with high biological activity are those having an bridge between Xaa¹⁸ and Xaa²² and/or between Xaa³⁰ and Xaa³⁴. A lysinoalanine is preferably formed between the side chains of amino acids Xaa¹⁸ and Xaa²¹, and/or Xaa¹⁸ and Xaa²², and/or Xaa³⁰ and Xaa³⁴, and/or Xaa³¹ and Xaa³⁴_{.}

The present inventors found that a cyclic peptide analog comprising a lysinoalanine between Xaa²⁶ and Xaa³⁰ of formula II (Xaa^{26-3o},II) has reduced biological activity. In view of the reduced activity of analog Xaa²⁶⁻³⁰,II, further surprising is the finding that a cyclic analog comprising a lysinoalanine between Xaa³⁰ and Xaa³⁴ of formula III (Xaa³⁰⁻³⁴,III) has a particularly high biological activity. Both these analogs have a lysinoalanine which involves amino acid position 30, and in both analogs Xaa³⁰ is dehydroalanine (Dha). Whereas the activity of GLP-1 analog Xaa²⁶⁻³⁰,II is reduced, the activity of GLP-1 analog Xaa³⁰⁻³⁴,III is at least equal to the activity of native GLP-1 and less susceptible to inhibition by a GLP-1 receptor antagonist (see Example 2). Thus, a preferred cyclic GLP-1 analog is His⁷-Ala⁸-Glu⁹-Gly¹⁰-Thr¹¹-Phe¹²-Thr¹³-Ser¹⁴-Asp ¹⁵-Val¹⁶-Ser¹⁷-Ser¹⁸-Tyr¹⁹-Leu²⁰-Glu²¹-Gly²²-Gln²³-Ala²⁴-Ala²⁵-Lys²⁶-Glu²⁷-Phe²⁸-Ile²⁹-Dha³⁰- Trp³¹-Leu³²-Val³³-Lys³⁴-Gly³⁵-Arg³⁶-Gly³⁷ wherein Dha³⁰ and Lys³⁴ form a lysinoalanine.

Other preferred cyclic peptide analogs are:
- His⁷-Ala⁸-Glu⁹-Gly¹⁰-Thr¹¹-Phe¹²-Thr¹³-Ser¹⁵Asp¹⁵-Val¹⁶-Ala¹⁷-Abu¹⁸-Tyr¹⁹-Leu²⁰-Glu²¹-Cys²²-Gln²³-Ala²⁴-Ala²⁵-Lys²⁶-Glu²⁷-Phe²⁸-Ile²⁹-Ala³⁰- Trp³¹-Leu³²-Val³³-Lys³⁴-Gly³⁵-Arg³⁶-Gly³⁷, wherein Abu¹⁸ and Cys²² form a methyllanthionine;
- His⁷-Ala⁸-Glu⁹-Gly¹⁰-Thr¹¹Phe¹²-Thr¹³-Ser¹⁴Asp¹⁵-Val¹⁶-Ala¹⁷-Ser¹⁸-Tyr¹⁹-Leu²⁰-Glu²¹-Gly²²-Gln²³-Ala²⁴-Ala²⁵-Lys²⁶-Glu²⁷-Phe²⁸-Ile²⁹-Abu³⁰- Trp³¹Leu³²-Val³³-Cys³⁴-Gly³⁵-Arg³⁶-Gly³⁷, wherein Abu³⁰ and Cys³⁴ form a methyllanthionine.
- His⁷-Ala⁸-Glu⁹-Gly^{l0}-Thr¹¹-Phe¹²-Thr¹³-Ser¹⁴-Asp¹⁵-Val¹⁶-Ala¹⁷-Abu¹⁸-Tyr¹⁹-Leu²⁰-Cys²¹-Gly²²-Gln²³-Ala²⁴-Ala²⁵-Lys²⁶-Glu²⁷-Phe²⁸-Ile²⁹-Ala³⁰- Trp³¹Leu³²-Val³³-Lys³⁴-Gly³⁵-Arg³⁶-Gly³⁷, wherein Abu¹⁸ and Cys²¹ form a methyllanthionine;
- His⁷-Ala⁸-Glu⁹-Gly¹⁰-Thr¹¹-Phe¹²-Thr¹³-Ser¹⁴-Asp¹⁵-Val¹⁶-Ser¹⁷-Ser¹⁸-Tyr¹⁹-Leu²⁰-Glu²¹Gly²²-Gln²³-Ala²⁴-Ala²⁵-Lys²⁶-Glu²⁷-Phe²⁷-Ile²⁹-Ala³⁰- Abu³¹-Leu³²-Val³³-Cys³⁴-Gly³⁵-Arg³⁶-Gly³⁷, wherein Abu³¹ and Cys³⁴ form a methyllanthionine;
- His⁷-Ala⁸-Glu⁹-Gly¹⁰-Thr¹¹-Phe¹²-Thr¹³-Ser¹⁴-Asp¹⁵-Val¹⁶-Ala¹⁷-Ser¹⁸-Tyr¹⁹-Leu²⁰-Glu²¹Gly²²-Gln²³-Ala²⁴-Ala²⁵-Lys²⁶-Glu²⁷-Phe²⁸-lIe²⁹-Dha³⁰- Trp³¹-Leu³²-Val³³-Lys³⁴-Gly³⁵-Arg³⁶-Gly³⁷, wherein Dha³⁰ and Lys³⁴ form a lysinoalanine;
- His⁷-Ala⁸-Glu⁹-Gly¹⁰-Thr¹¹-Phe¹²-Thr¹³-Ser¹⁴-Asp¹⁵-Val¹⁶-Ala¹⁷-Abu¹⁸-Tyr¹⁹-Leu²⁰-Cys²¹Gly²²-Gln²³-Ala²⁴-Ala²⁵-Lys²⁶-Glu²⁷-Phe²⁸-Ile²⁹-Ala³⁰- Trp³¹-Leu³²-Val³³-Lys³⁴-Gly³⁵-Arg³⁶-Gly³⁷, wherein Abu¹⁸ and Cys²¹ form a methyllanthionine;
- His⁷-Ala⁸-Glu⁹-Gly¹⁰-Thr¹¹-Phe¹²-Thr¹³-Ser¹⁴-Asp¹⁵-Val¹⁶-Ala¹⁷-Ser¹⁸-Tyr¹⁹-Leu²⁰-Glu²¹Gly²²-Gln²³-Ala²⁴-Ala²⁵-Lys²⁶-Glu²⁷-Phe²⁸-Ile²⁹-Ala³⁰- Abu³¹-Leu³²-Val³³-Cys³⁴-Gly³⁵-Arg³⁶-Gly³⁷, wherein Abu³¹ and Cys³⁴ form a methyllanthionine.

In one embodiment, a cyclic peptide analog according to the invention has one enzymatically formed cyclic structure between the side chains of two amino acids. However, a cyclic peptide analog may also have more than one of such cyclic structures, such as two, three of four cyclic structures. Preferably, a cyclic peptide analog has one, two or three intramolecular linkages. If more than one bridge is present in a cyclic peptide analog according to the invention all of these bridges may be of one type, i.e. all linkages are either a lanthionine and/or a methyllanthionine and thus have the general formula I, or all linkages are lysinoalanines and thus have the general formula II or III. It is, however, also possible that a combination of one or more lanthionines and/or methyllanthionines and one or more lysinoalanine are present within one cyclic peptide analog.

Cyclic peptide analogs according to the present invention are advantageously produced enzymatically using enzymes that are capable of dehydrating serine and threonine. The present inventors found that Ser¹⁷ of GLP-1, GIP, Exendin3, Exendin4, PHM27 and Peptide His Ile is sensitive to dehydration by such enzymes. It is therefore advantageous to substitute the serine at this position in a cyclic analog by an amino acid other than serine or threonine. That way, this serine cannot be dehydrated to dehydroalanine and subsequently interfere with bridge formation between the side chains of two amino acids at other positions. Ser¹⁷ is preferably substituted by an alanine. Furthermore, it is particularly advantageous to substitute Ser¹⁷ by another amino acid if a bridge is to be formed between the side chains of two amino acids located close to position 17, because in that case the risk of interference of a dehydrated Ser¹⁷ with bridge formation is most prominent. Amino acids at positions 10-16 and 18-25 are considered to be located close to position 17. Therefore, Ser¹⁷ is preferably substituted by another amino acid if an intramolecular linkage within a cyclic peptide analog is formed between the side chain of at least one amino acid at position 10-16 or 18-25.

In one embodiment, Ala⁸ in a cyclic peptide analog of a GLP-1 (related) peptide is replaced by another amino acid. It is well known that DPP-IV cleaves GLP-1 and GLP-1 related peptides between amino acids at positions 8 and 9. Substitution of Ala⁸ has been demonstrated to slightly enhance resistance of cleavage of analogs of GLP-1 by DPP-IV. Ala⁸ can be substituted by any amino acid, for example by glycine, alpha-aminoisobutyric acid, serine, threonine, or a D-amino acid such as D-alanine. Preferably Ala⁸ is replaced by a dehydrobutyrine (Dhb), a glycine or a D-amino acid. Dehydrobutyrine is naturally the most occurring dehydroamino acid. Dhb is however, known in the art to be unstable because it is susceptible to hydration and intrinsic reactivity), and it would therefore be expected to be unsuitable for stabilizing a cyclic peptide analog according to the invention. The present inventors surprisingly found that in particular cyclic peptide analogs that have a dehydrobutyrine at position 8 (Dhb⁸) have enhanced resistance to cleavage by DPP-IV, without resulting in an unstable analog. More preferably, therefore, Ala⁸ in a cyclic peptide analog is a dehydrobutyrine.

Particularly preferred cyclic peptide analogs have both enhanced resistance to cleavage by DPP-IV between amino acids 8 and 9 and enhanced resistance to cleavage by NEP 24.11 in the C-terminal part of the analog, i.e. between amino acid positions 13 and the C-terminus, as compared to their linear counterparts. Therefore, preferred cyclic peptide analogs have an amino acid other than Ala⁸ to enhance resistance to DPP-IV cleavage and at least one intramolecular linkage between the side chains of two amino acids that are located between amino acid position 15 and the C-terminus of the analog to enhance C-terminal proteolytic resistance. An example of such a preferred cyclic peptide analog is an analog wherein Xaa⁸ is a dehydrobutyrine, a glycine or a D-amino acid and which comprises an enzymatically formed intramolecular linkage between Xaa¹⁸ and Xaa²². Preferably, Xaa¹⁷ in said analog is alanine. Another example of a preferred cyclic peptide analog is an analog wherein Xaa⁸ is a dehydrobutyrine, a glycine or a D-amino acid and which comprises an enzymatically formed intramolecular linkage between Xaa³⁰ and Xaa³⁴, preferably wherein Xaa¹⁷ is alanine. Said intramolecular linkage in both preferred analogs is preferably an lysinoalanine.

Intramolecular linkages between the side chains of two amino acids in a cyclic peptide are enzymatically formed. This is for instance achieved using enzymes of lactic acid bacteria which are involved in the production of lantibiotics. Lantibiotics and lantipeptides (lantionine containing peptides which lack antimicrobial acitivity) are small peptides containing internal bridges resulting from the formation of (methyl)lanthinione or lysinoalanyl residues (reviewed in McAuliffe et al., FEMS Microbiol. Rev. 25,:285-308 (2001). Lanthionine (Lan) and methyllanthionine (MeLan) result from the dehydration of serine to dehydroalanine (Dha) and of threonine to dehydrobutyrine (Dhb), respectively, and thioether bond formation resulting from the interaction of these amino acids with a cysteine residue within the same peptide. Lysinoalanine is formed upon interaction of dehydroalanine with a serine residue. Well known examples of lantibiotics are nisin, subtilin, Pep5, and epiderminin.

Lantibiotics are ribosomally synthesized as inactive prepeptides, containing an amino terminal leader peptide and a carboxy terminal propeptide. The leader peptide is necessary for the interaction of the lantibiotic precursor with lantibiotic enzymes. The leader peptide is proteolytically cleaved from the propeptide either inside the producing cell, or during or after transport out of the cell. The lantipeptide biosynthetic genes are clustered together and are given the locus name *lan. LanA* is the gene encoding the lantibiotic prepeptide, *lamB* is the gene encoding an enzyme responsible for serine or threonine dehydration and *lanC* is the gene encoding an enzyme responsible for cycle formation. In some cases, the activities of LanB and LanC are joined in a bifunctional enzyme encoded by *lanM. lanP,* and *lanT,* genes encode enzymes that are involved in processing and/or transport of prepeptides.

Cyclic peptide analogs are advantageously prepared using a host cell comprising lantiopeptide biosynthetic enzymes. Accordingly, the invention provides a method for the enzymatic preparation of a cyclic peptide analog according to the invention, comprising:
a) providing a host cell comprising:
   - a nucleic acid molecule comprising a first nucleic acid fragment encoding an N-terminal leader peptide found with the prepeptide of a lantibiotic and a second nucleic acid fragment encoding a peptide analog, whereby said first and second fragment are within the same open reading frame of said nucleic acid molecule;
   - a nucleic acid sequence encoding an enzyme capable of dehydrating serine and/or threonine;
   - optionally a nucleic acid sequence encoding a transporter protein; and
   - a nucleic acid sequence encoding an enzyme capable of inducing lanthionine and/or methyllanthionine formation if the enzymatically formed intramolecular linkage has the general formula I;
b) allowing for the translation of said first nucleic acid;
c) harvesting said peptide analog;
d) inducing lysinoalanine bridge formation by exposing the peptide to a pH of between 8 and 11 if the enzymatically formed intramolecular linkage has the general formula II or III.

If the enzymatically formed intramolecular linkage has the general formula I step d) is omitted.

Any leader peptide found with the prepeptide of a lantibiotic is suitable for use in a method for the preparation of cyclic peptide analogs according to the invention. Examples of such leader peptides are the leader peptides of nisin, subtilin, Pep5, and epiderminin. In a preferred embodiment, a nisin leader peptide is used.

The enzyme capable of dehydrating serine and/or threonine preferably is a LanB, such as NisB, PepB, SpaB, or a functional equivalent thereof. The enzyme capable of inducing lanthionine and/or methyllanthionine formation preferably is a LanC, such as NisC, PepC, SpaC, or a functional equivalent thereof. In one embodiment, dehydration of serine and/or threonine and lanthionine and/or methyllanthionine formation are induced by the same enzyme, a LanM, such as, CinM, LtnM, LctM, or a functional equivalent thereof.

Lanthionine formation between dehydroalanine and cysteine is energetically possible at room temperature and can also occur spontaneously, without the involvement of a LanC or LanM enzyme. Therefore, in one embodiment, a method is provided for the enzymatic preparation of a cyclic peptide analog according to the invention comprising at least one lanthionine, comprising:
a) providing a host cell comprising:
   - a nucleic acid molecule comprising a first nucleic acid fragment encoding an N-terminal leader peptide found with the prepeptide of a lantibiotic and a second nucleic acid fragment encoding a peptide analog, whereby said first and second fragment are within the same open reading frame of said nucleic acid molecule;
   - a nucleic acid sequence encoding an enzyme capable of dehydrating serine and/or threonine;
   - (optionally) a nucleic acid sequence encoding a transporter protein; and
b) allowing for the translation of said first nucleic acid;
c) harvesting said peptide analog.

For the purpose of harvesting a cyclic peptide analog or a peptide analog that has not yet been cyclized it is especially preferred that a transporter protein is present if the host cell is a Gram-positive bacterium. Preferably said transporter protein is a LanT, such as NisT, or a functional equivalent thereof. In *Escherichia coli* the presence of a transporter is not required and harvesting of the peptide analog preferably follows disruption of the cells.

Lysinoalanine formation between a dehydroalanine and a lysine is possible without the involvement of an enzyme. Spontaneous lysinoalanine formation requires an environment with a pH of between 8 and 11. Therefore, in one embodiment, a harvested analog of GLP-1 (related) peptide is exposed to a pH of between 8 and 11 to induce cyclization. Preferably said analog is exposed to a pH of between 10 and 11. Exposure of an analog to such pH is for example achieved by increasing the pH in an aqueous solution comprising the analog using a suitable alkaline agent, e.g. NaOH or ammonia. The analog may be incubated in the presence of about 0,25% (w/v) ammonia. Recently, an enzyme capable of inducing lysinoalanine formation during the synthesis of cinnamycin was identified, which was called Cinorf7 (Okesli A. et al. J. Am. Chem. Soc. 2011, 133: 13753-60). It has thus become possible to enzymatically induce lysinoalanine formation following dehydration of a serine and/or threonine. Therefore, in one embodiment, a host cell is used which further comprises a nucleic acid sequence encoding an enzyme capable of inducing lysinoalanine formation. Said enzyme capable of inducing lysinoalanine bridge formation is preferably Cinorf7 or a functional equivalent of Cinorf7.

A host cell used in a method for the preparation of a cyclic analog according to the invention is preferably a Gram-positive prokaryote, a Gram-negative prokaryote or an eukaryote. Examples of suitable host cells include lactic acid bacteria, such as *Lactococcus lactis, Bacillus subtilis, Staphylococcus epidermis* and the Gram-negative bacterium *E*. *coli.*

As detailed above, a lanthionine or a methyllanthionine is formed between the side chains of a dehydrated serine or threonine, i.e. a dehydroalanine or a dehydrobutyrine respectively, and a cysteine. Therefore, a nucleic acid sequence encoding a peptide analog of GLP-1 or of a GLP-1 related peptide, which when cyclised comprises at least one lanthionine or methyllanthionine, needs to have a nucleic acid codon encoding a serine (TCT, TCC, TCA, TCG, AGT or AGC) or a threonine (ACT, ACC, ACA or ACG) and a nucleic acid codon encoding a cysteine (TGT or TGC) at the two amino acid positions within the peptide analog which will form the lanthionine or methyllanthionine. The same applies for a lysinoalanine. A lysinoalanine is formed between the side chains of a dehydrated serine, i.e. a dehydroalanine, and a lysine. Therefore, a nucleic acid sequence encoding a peptide analog of a GLP-1 (related) peptide, which when cyclised comprises a lysinoalanine, needs to have a nucleic acid codon encoding a serine (TCT, TCC, TCA, TCG, AGT or AGC) and a nucleic acid codon encoding a lysine (AAA or AAG) at the two amino acid positions within the peptide analog which will be part of the lysinoalanine.

A cyclic structure can be formed between the side chains of two (dehydrated) amino acids that are present in GLP-1 or a GLP-1 related peptide, or between the side chains of two amino acids, one or both of which are inserted into a GLP-1 or related peptide analog by replacement of the amino acid present in the native GLP-1 or related analog. Thus, a serine, threonine, cysteine, or lysine that, after dehydration of serine/threonine, will be part of a lanthionine, a methyllanthionine or a lysinoalanine may be originally present in GLP-1 or related peptide. However, one or both of the amino acid may also be inserted into a GLP-1 or related peptide analog by replacement of amino acids at the positions intended to be part of the lanthionine, methyllanthionine, or lysinoalanine.

Preferred are cyclised peptides of the analogs depicted in table 1. Therefore, in a preferred embodiment, the second nucleic acid fragment located on the nucleic acid molecule of the host cell encodes a peptide analog selected from table 1.

**Table 1. Preferred cyclic peptide analogs according to the invention. Amino acid substitutions in comparison with wild type GLP-1 are underlined.**

| name | sequence |
|---|---|
| | |
| GLP1-M6 | HAEGTFTSDVATYLECQAAKEFIAWLVKGRG |
| GLP1-M9 | HAEGTFTSDVASYLEGQAAKEFITWLVCGRG |
| GLP1-LAL(26-30) | HAEGTFTSDVASYLEGQAAKEFISWLVKGRG |
| GLP1M1LAL (30-34) | HAEGTFTSDVASYLEGQAAKEFISWLVKGRG |
| GLP1-M8c | HAEGTFTSDVATYLCGQAAKEFIAWLVKGRG |
| GLP1-M10c | HAEGTFTSDVASYLEGQAAKEFIATLVCGRG |

For therapeutic application, preferably a cyclic peptide analog is selected which has a enhanced biological activity as compared to the linear counterpart GLP-1 (related) peptide. In one embodiment, a method for the preparation of a cyclic peptide analog therefore further comprises selecting a cyclic peptide analog that has enhanced biological activity as compared to the linear counterpart GLP-1 (related) peptide. Suitable processes for selecting a cyclic peptide analog that enhanced biological activity include, for example, measuring biological activity in an *in vitro* assay system using said cyclic peptide analog and a competitor, such as the linear counterpart GLP-1 (related) peptide. In one embodiment, said biological activity measured is extracellular cAMP production, intracellular cAMP production, or insulin production.

An *in vitro* cell functional assay is for instance performed using a reporter cell line, such as RIN-5F cells, that express the receptor that is specifically bound by the cyclic peptide analog and its linear counterpart, i.e. GLP1R or the receptor for a GLP-1 related peptide. For instance, increases in cAMP levels caused by enhanced luciferase reporter gene expression induced by a cyclic peptide analog and a competitor, such as its linear counterpart, can be measured. cAMP levels are for instance measured via competition-based ELISA. It is also possible to perform competition assays in such reporter cell lines. In that case, cells are incubated with a cyclic peptide analog or its linear counterpart in the presence of a receptor antagonist.

It is also preferred that a cyclic peptide analog is selected which has enhanced resistance to proteolytic cleavage as compared to the linear (non-cyclised) counterpart GLP-1 (related) peptide. In one embodiment, a method for the preparation of a cyclic peptide analog therefore further comprises selecting a cyclic peptide analog that has enhanced resistance to proteolytic cleavage as compared to the linear counterpart. Suitable processes for selecting a cyclic peptide analog that has enhanced resistance to proteolytic cleavage include, for example, measuring *in vitro* plasma stability, and direct measurement of degradation of an analog and its linear counterpart by proteolytic enzymes, for example recombinant, DPP-IV and/or NEP 24.11.

*In vitro* plasma stability is for instance measured by incubating a both cyclic peptide analog and its linear counterpart GLP-1 (related) peptide in human serum. The half life of the analog and linear counterparts can then be determined by measuring the concentration of intact analog and linear counterpart GLP-1 (related) peptide at several time points following incubation in the plasma. For instance, mass spectrometry or HPLC can be used following extraction of analog and linear counterpart GLP-1 (related) peptide from the plasma. Direct measurement of degradation of an analog and its linear counterpart by DPP-IV or NEP 24.11 may be achieved by incubating a cyclic peptide analog and its linear counterpart with, for instance recombinant, DPP-IV or NEP 24.11. The amount of intact and degraded peptide can be analysed by, for instance, HPLC.

Also provided is a pharmaceutical composition comprising a cyclic peptide analog according to the invention, or a pharmaceutically acceptable salt or complex thereof, and a pharmaceutically acceptable carrier, diluent and/or excipient. In another embodiment, a pharmaceutical composition comprises more than one cyclic peptide analog according to the invention, for instance, 2, 3, 4, or 5 cyclic peptide analogs.

As used herein, "pharmaceutically acceptable salt" refers to salts that retain the desired activity of the peptide or equivalent compound, but preferably do not detrimentally affect the activity of the peptide or other component of a system, which uses the peptide. Examples of such salts are acid addition salts formed with inorganic acids, for example, hydrochloric acid, hydrobromic acid, sulfuric acid, phosphoric acid, nitric acid, and the like. Salts may also be formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, pamoic acid, alginic acid, polyglutamic acid, and the like. Salts may be formed with polyvalent metal cations such as zinc, calcium, bismuth, barium, magnesium, aluminium, copper, cobalt, nickel and the like or with an organic cation formed from N,N'-dibenzylethylenediamine or ethylenediamine, or combinations thereof (e.g., a zinc tannate salt). The non-toxic, physiologically acceptable salts are preferred.

The salts can be formed by conventional means such as by reacting the free acid or free base forms of the product with one or more equivalents of the appropriate acid or base in a solvent or medium in which the salt is insoluble, or in a solvent such as water which is then removed *in vacuo* or by freeze-drying, or by exchanging the cations of an existing salt for another cation on a suitable ion exchange resin.

The term "pharmaceutically acceptable complex" of the peptides according to the invention is used herein to refer to complex compounds formed with certain, for instance organic materials, endowing the peptides with a retarded activity. Typical representatives of these compounds are gelatines, carboxymethylcelluloses, alginic acid esters, poly(fluoroethinephosphates), amino acid polymers or other polymers and copolymers.

Examples of suitable carriers for instance comprise keyhole limpet haemocyanin (KLH), serum albumin (e.g. BSA or RSA) and ovalbumin. In one preferred embodiment said suitable carrier comprises a solution, like for example saline.

A pharmaceutical composition according to the invention may also contain an adjuvant. Illustrative of the adjuvants which can be incorporated in tablets, capsules and the like are the following: a binder such as gum tragacanth, acacia, corn starch or gelatin; an excipient such as microcrystalline cellulose; a disintegrating agent such as corn starch, pregelatinized starch, alginic acid and the like; a lubricant such as magnesium stearate; a sweetening agent such as sucrose, lactose or saccharin; a flavoring agent such as peppermint, oil of wintergreen or cherry. When the dosage unit form is a capsule, it may contain, in addition to materials of the above type, a liquid carrier such as fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

A pharmaceutical composition according to the invention is preferably suitable for human use. Preferably, the composition comprises a cyclic peptide analog, the amino acid sequence of which is shown in table 1.

Also provided is a cyclic peptide analog according to the invention for use as a medicament.

In one embodiment, the invention provides a method for the treatment or prophylaxis of a metabolic disease, such as diabetes mellitus, metabolic syndrome, obesity, comprising administering to a subject in need thereof a therapeutically effective dose of a cyclic peptide analog according to the invention having GLP-1 activity or activity of a GLP-1 related peptide, or of a pharmaceutical composition according to the invention. Preferably said subject is a human. Also provided is a pharmaceutical composition according to the invention or a cyclic peptide analog according to the invention for use in a method for the treatment or prophylaxis of a metabolic disease, such as diabetes mellitus, metabolic syndrome, or obesity.

Another useful application of cyclic peptide analogs according to the invention is in cardiovascular disease. The invention therefore also provides a method for the treatment or prophylaxis of a cardiovascular disease, such as ischaemia/reperfusion injury, hypertensive-related heart failure, or coronary artery disease, comprising administering to a subject in need thereof a therapeutically effective dose of a cyclic peptide analog according to the invention having GLP-1 activity or activity of a GLP-1 related peptide, or of a pharmaceutical composition according to the invention. Preferably said subject is a human. Also provided is a pharmaceutical composition or cyclic peptide analog according to the invention for use in a method for the treatment or prophylaxis of a cardiovascular disease, such as ischaemia/reperfusion injury, hypertensive-related heart failure, or coronary artery disease.

Yet another useful application of cyclic peptide analogs according to the invention is in neurological or neurodegenerative disease. The invention therefore also provides a method for the treatment or prophylaxis of a neurological or neurodegenerative disease, such as Alzheimer's disease, Parkinson's disease, Huntington's disease, stroke or peripheral sensory neuropathy, comprising administering to a subject in need thereof a therapeutically effective dose of a cyclic peptide analog according to the invention having GLP-1 activity or activity of a GLP-1 related peptide, or of a pharmaceutical composition according to the invention. Preferably said subject is a human. Also provided is a pharmaceutical or a cyclic peptide analog for use in a method for the treatment or prophylaxis of a neurological or neurodegenerative disease, such as Alzheimer's disease, Parkinson's disease, Huntington's disease, stroke or peripheral sensory neuropathy.

Cyclic peptide analogs according to the invention may be utilized in compositions such as tablets, capsules or elixirs for oral administration, suppositories for rectal administration, sterile solutions or suspensions for enteral, parenteral or intramuscular administration, and the like. The compounds of the invention will normally be administered orally, intravenously, subcutaneously, buccally, rectally, dermally, nasally, tracheally, bronchially, by any other parenteral route or via inhalation, in a pharmaceutically acceptable dosage form.

Sterile compositions for injection can be formulated according to conventional pharmaceutical practice by dissolving or suspending the analog in a vehicle such as water for injection, a naturally occurring vegetable oil like sesame oil, coconut oil, peanut oil, cottonseed oil, etc., or a synthetic fatty vehicle like ethyl oleate or the like. Buffers, preservatives, antioxidants and the like can be incorporated as required.

The cyclic peptide analogs according to the invention can be administered to patients (animals and human) in need of such treatment in dosages that will provide optimal pharmaceutical efficacy. Although the dose will vary from patient to patient depending upon the nature and severity of disease, the patient's weight, special diets then being followed by a patient, concurrent medication, and other factors which those skilled in the art will recognize, the dosage range will generally be about 1 to 1000 mg per patient per day which can be administered in single or multiple doses. The dosage range can be about 2.5 to 250 mg per patient per day; such as about 2.5 to 75 mg per patient per day.

### Brief description of the drawings

Figure 1. Sequences alignment of GLP-1 and GLP-1 related peptides showing mutual homology. In the bottom line conserved residues are indicated.

Figure 2. Proteolytic resistance of a lysinoalanine-stabilized peptide analog. HPLC purification of GLP-1 fragment (29-36) fused to the nisin leader : nisin leader - I Dha W L V K G R , wherein a lysinoalanine was enzymatically induced between the Dha and K, treated with trypsin and carboxypeptidase Y (cpY).
Peak I: linear peptide showing no protection against trypsin and cpY
Peak II: lysinoalanine variant showing protection against trypsin and cpY
Peak III: lysinoalanine variant showing protection against trypsin and cpY
Peak IV: linear peptide showing cleavage by trypsin and protection against cpY cleavage.

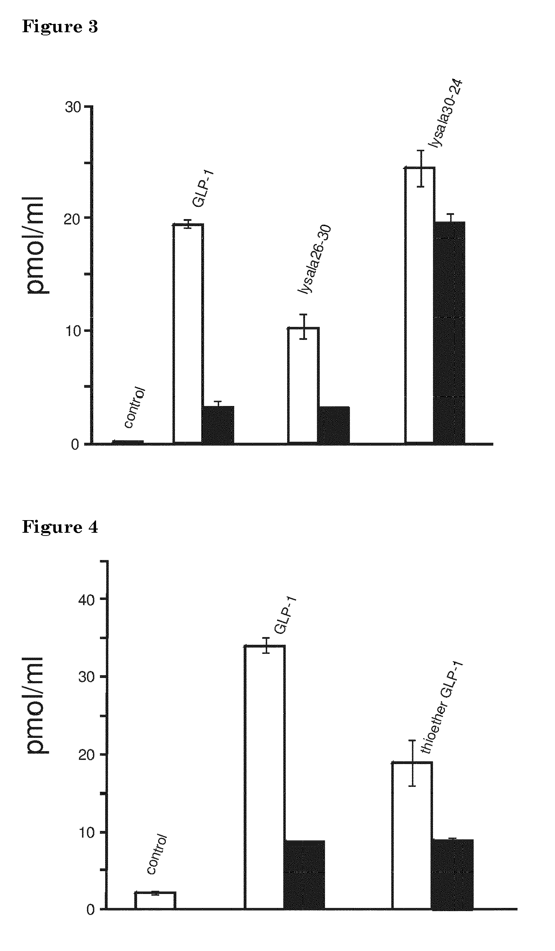

Figure 3. Activity of lysinoalanine-stabilized GLP-1 analogs in the absence and presence of the antagonist exendin-9 as compared to native GLP-1. Open bars from left to right: control (absence of GLP-1 or analog), GLP-1, GLP-1 lysinoalanine 26-30, GLP-1 lysinoalanine 30-34. Filled bars: GLP-1 and GLP-1 cyclized variants with pre-incubated exendin-9. GLP-1 lysinoalanine 30-34 is more active than native GLP-1 and binds more tightly since the antagonist has no effect and is apparently displaced.

Figure 4. Activity of 18-21 thioether stabilized GLP-1 as compared to native GLP-1. Open bars from left to right: control (absence of GLP-1 or analog); GLP-1, GLP-1 thioether bridge 18-21. Filled bars: GLp-1 and GLP-1 thioether bridge 18-21 with pre-incubated exendin-9.

The invention is further explained in the following examples. These examples do not limit the scope of the invention, but merely serve to clarify the invention.

### EXPERIMENTAL SECTION

### Example 1: Lysinoalanine-bridged GLP-1 peptide is resistant to peptidase-mediated breakdown.

In this example one serine in a GLP-1 fragment was enzymatically converted to a dehydroalanine. Lysinoalanine formation was induced between this dehydroalanine and a lysine present in the GLP-1 fragment. The dehydrated peptide was produced by *Lactococcus lactis* containing a nisin dehydratase responsible for the conversion. By incubation with high pH a lysinoalanine was formed. The lysinoalanine-bridged GLP-1 peptide was shown to be resistant against cleavage by trypsin and carboxypeptidase Y.

### Methods

Production of peptide in *Lactococcus lactis* and characterization of the peptide were performed as described in Plat et al. (Polymers 2011, 3, 1282-1296). In brief, a *Lactococcus* strain containing pIL3BTC, encoding the nisin dehydratase NisB, the nisin cyclase NisC and the nisin export system NisT, and a pNZ8048 derived plasmid encoding the nisin leader peptide fused to a sequence encoding a GLP-1 fragment (29-36) resulting in: nisin leader - I S W L V K G R. After inducing dehydration the peptide was harvested from the medium and characterized.

### Results

The fusion peptide was successfully secreted and dehydrated. A high amount of peptide was produced and isolated. After one hour incubation in the presence of 0.25 % ammonia (w/v) at 60 °C more than 80% was converted to lysinoalanine. This was noticed by HPLC by a drop in the 254 nm signals. A peak at 254 nm represents dehydroalanine. After formation of a lysinoalanine bridge, dehydroalanine is no longer present in the peptide, which results in a decrease of peak intensity at 254 nm. Confirmation of presence of the lysinoalanine was done by mass spectrometry, trypsin cleavage and incubation with carboxypeptidase Y (cpY).

After overnight incubation of the purified peptide containing a lysinoalanine at RT in 0.25 % ammonia (w/v) the sample was applied on a C18 column (Alltech, alltima analytical C18 column 5 µm). Peaks were collected and analyzed by mass spectrometry. Different conformations of the peptide were incubated with trypsin and cpY (Figure 2).

Lysinoalanine variants of GLP-1 were formed which have been separated by HPLC. The lysinoalanine variants provide protection against breakdown by trypsin and carboxypeptidase Y.

### Example 2: Enhanced receptor binding and activity of lysinoalanine-bridged GLP-1 by stabilizing the C-terminal alpha helix structure

The N-terminal part of GLP-1 is involved in signal transduction whereas the C-terminal part of GLP-1 binds to the receptor in an alpha helical structure. In this experiment the alpha helical structure in GLP-1 is stabilized by a lysinoalanine bridge. Since a lysinoalanine bridge is larger than a thioether bridge these bridges may be complementary in suitability depending on the distance of the positions to be bridged. Concomitantly the lysinoalanine will provide protection against peptidase action.

Serine17 in GLP-1 is readily dehydrated by the lantibiotic dehydratase, NisB. The resulting dehydroalanine may cause instability due to its reactivity. In addition it would complicate analysis on the position of the lysinoalanine. Therefore Ser¹⁷ was mutated to an alanine. Ala³⁰ on the other hand was mutated to a serine to allow two lysinoalanine bridges to be formed, one from lysine 26 to dehydroalanine 30, and alternatively one from dehydroalanine 30 to lysine 34. Lysines involved in lysinoalanine formation are K²⁶ in GLP-1M1lal and K³⁴ in GLP-1M1lalb in the sequences below.

Sequence of GLP-1 and GLP-1 analogs synthesized in this experiment:

| | |
|---|---|
| | 7 10 15 20 25 30 35 |
| GLP-1: | HAEGTFTSDVSSYLEGQAAKEFIAWLVKGRG |
| GLP-1M1c: | HAEGTFTSDVASYLEGQAAKEFIAWLVKGRG |
| GLP-1M1lal: | HAEGTFTSDVASYLEGQAAKEFI(Dha)WLVKGRG |
| GLP-1M1lalb: | HAEGTFTSDVASYLEGQAAKEFI(Dha)WLVKGRG |

### Methods

### Peptide synthesis.

*Lactococcus lactis* containing plasmid pIL3BTC encoding the nisin modification enzymes NisB, NisC and the nisin transporter NisT was transformed with a pNZ8048 derived plasmid encoding a fusion peptide composed of the N-terminal nisin leader peptide and GLP-1 analogs. In peptide analogs with a dehydroalanine in position 30 lysinoalanine formation between either Dha³⁰ and Lys²⁶ or Dha³⁰ and Lys³⁴ was induced by high pH treatment, i.e. incubation in the presence of 0.25 % (w/v) ammonia at 60 °C. Each GLP-1 analog was subsequently purified by HPLC.

### General methods

C18 column was from Alltech, alltima analytical C18 column 5 µm. A gradient of 10% (5 min) to 40% (35 min) acetonitrile in 0.1% trifluoroacetic acid was applied when performing HPLC. Plasmids, *Lactococcus lactis* strain, culture conditions, genetic engineering, HPLC and Maldi TOF mass spectrometry are described in Plat et al. (Polymers 2011, 3, 1282-1296).

### Activity testing

The Rin-5F cell-line RIN-5F (pancreas Beta cell line) was obtained from ATCC (number: CRL-11605 ™). cAMP ELISA kit was obtained from Enzo Life Sciences.

Induction of cAMP production in RIN-5F cells after challenge with GLP-1 or a GLP-1 analog was performed as follows:
- 10⁶ Rin-5F cells were seeded per well (6-wells plates).
- Cells were incubated overnight with medium lacking FBS.
- Cells were challenged with:
   1) medium (negative control), Exendin-4 (agonist), Exendin-9 (antagonist), human GLP-1 (7-36) (positive control) or GLP-1 analogs GLP-1M1c, GLP-1M1lal and GLP-1M1lalb (see above) for 10 minutes at 100 nM.
   2) GLP-1 or cyclic GLP-1 analogs with pre-incubated Exendin-9 (10 min at 100 nM).
- The cAMP content of medium was determined by competitive ELISA.

### Results

Results are shown in Figure 3. GLP-1 induces cAMP release. cAMP release is inhibited by the antagonist exendin-9, which consists of GLP-1 lacking the N-terminal residues 7 and 8. The cyclic GLP-1 analog with Ser17Ala and lysinoalanine Dha³⁰-Lys³⁴ is slightly more active than wild type GLP-1 and is not antagonized by preincubation with exendin-9. The cyclic GLP-1 analog with Ser17Ala and lysinoalanine Lys²⁶-Dha³⁰ has reduced activity but its enhanced stability *in vivo* may lead to a net improvement.

These data demonstrate that a cyclic GLP-1 analog with Ser17Ala and lysinoalanine 30-34 is more active than wild type GLP-1 and has strongly improved receptor binding, in addition to stabilization of the alpha helix structure as demonstrated in Example 1.

### Example 3. Stabilizing GLP-1 by the enzymatic introduction of a thioether bridge.

In this example a thioether bridged is formed in GLP-1 at position 18-21.

### Methods

Production of peptides in *Lactococcus lactis* and characterization of the peptide were performed as described in Plat et al. (Polymers 2011, 3, 1282-1296). In brief, *Lactococcus lactis* containing plasmid pIL3BTC encoding the nisin modification enzymes NisB, NisC and the nisin transporter NisT was transformed with a pNZ8048 derived plasmid encoding a fusion peptide composed of the N-terminal nisin leader peptide and GLP-1 variant HAEGTFTSDVATYLCGQAAKEFIAWLVKGRG. After induction of dehydration and cyclisation the produced cyclic GLP-1 analog was harvested from the culture supernatant and characterized. C18 column was obtained from Alltech, alltima analytical C18 column 5 µm. Activity testing was performed as described in example 2.

### Results

An active, stabilized 18-21 thioether bridged GLP-1 analog is produced (figure 4).

## Claims

1. A cyclic peptide analog of GLP-1 or of a GLP-1-related peptide, comprising at least one enzymatically formed intramolecular linkage between the side chains of two amino acids.

2. Cyclic peptide analog according to claim 1, which has enhanced biological activity as compared to the linear counterpart GLP-1 or GLP-1 related peptide.

3. Cyclic peptide analog according to claim 1 or 2, wherein said GLP-1-related peptide is selected from the group consisting of GLP-2 (PDB accession no. 2L63_A), glucagon (PRF accession no. 570104A), GIP (PDB accession no. 2B4N_A), exendin-4 (GenBank accession no. AAB22006), exendin-3 (Swiss-Prot accession no. P20394), VIP (PRF accession no. 0601216A), PACAP27, PACAP38 (GenBank accession no. AAB20402), helospectin (Swiss-Prot accession no. P04203), helodermin (Swiss-Prot accession no. P04204), PHM27 (PRF accession no. 1010243A), peptide His Ile (PRF accession no. 1011215A) and GRF (GenBank accession no. CAA24956).

4. Cyclic peptide analog according to any one of claims 1-3, comprising an intramolecular linkage of the general formula 1: 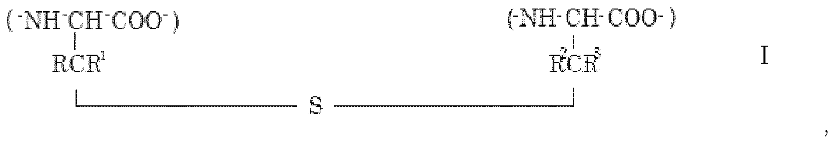 wherein R¹, R² and R³ are independently selected from -H, a lower (e.g. C₁-C₁₀) alkyl or aralkyl group, preferably wherein R¹, R² and R³ are independently selected from H and CH₃.

5. Cyclic peptide analog according to any one of claims 1-4, comprising an intramolecular linkage of the general formula II or III: 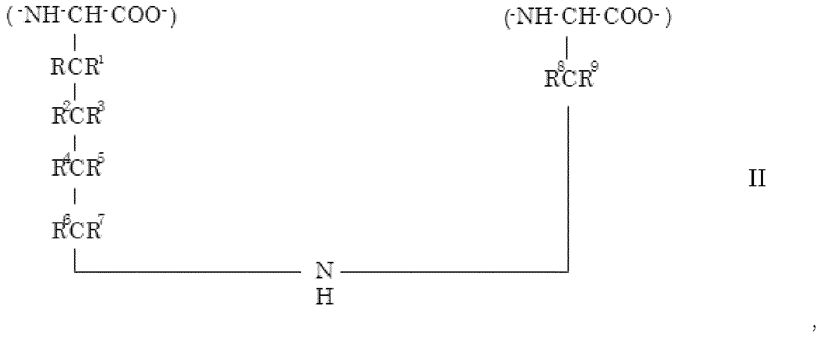 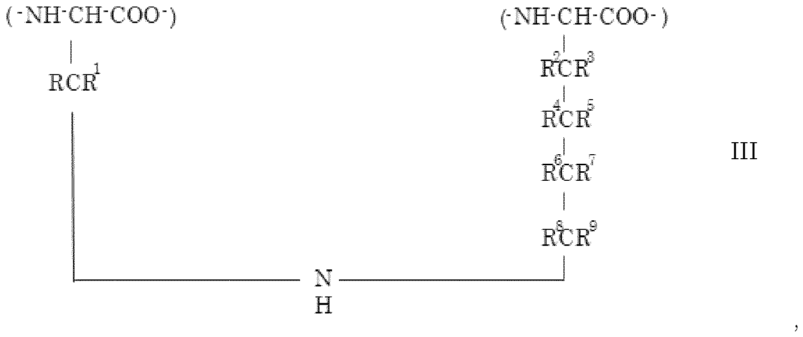 wherein R¹, R², R³, R⁴, R⁵, R⁶, R⁷, R⁸ and R⁹ are independently selected from -H, a lower (e.g. C₁-C₁₀) alkyl or aralkyl group, preferably wherein R¹, R², R³, R⁴, R⁵, R⁶, R⁷, R⁸ and R⁹ are independently selected from H and CH₃.

6. Cyclic peptide analog according to any one of claims 1-5, which comprises a thioether bridge and/or lysinoalanine bridge between the side chains of the amino acids corresponding to positions Xaa¹⁸ and Xaa²¹, and/or Xaa¹⁸ and Xaa²², and/or Xaa²⁴ and Xaa²⁸, and/or Xaa³⁰ and Xaa³⁴, and/or Xaa³¹ and Xaa³⁴ as defined in figure 1.

7. Cyclic peptide analog according to any one of claims 1-6, wherein Xaa¹⁷ as defined in figure 1, is Ala.

8. Cyclic peptide analog according to any one of claims 1, 2 and 4-7, which is an analog of GLP-1.

9. Cyclic peptide analog according to claim 8, of which the amino acid sequence before cyclisation is depicted in table 1.

10. Pharmaceutical composition comprising a cyclic peptide analog according to any one of claims 1 to 9, or a pharmaceutically acceptable salt or complex thereof, and a pharmaceutically acceptable carrier, diluent and/or excipient.

11. A cyclic peptide analog according to any one of claims 1-9 for use as a medicament.

12. Pharmaceutical composition according to claim 10 for use in a method for the treatment or prophylaxis of a cardiovascular disease, such as ischaemia/reperfusion injury, hypertensive-related heart failure, or coronary artery disease, a metabolic disease, such as diabetes mellitus, metabolic syndrome, or obesity, or a neurological or neurodegenerative disease, such as Alzheimer's disease, Parkinson's disease, Huntington's disease, stroke or peripheral sensory neuropathy.

13. A method for the enzymatic preparation of a cyclic peptide analog according to any one of claims 1-9, comprising:
a) providing a host cell comprising:
- a nucleic acid molecule comprising a first nucleic acid fragment encoding an N-terminal leader peptide found with the prepeptide of a lantibiotic and a second nucleic acid fragment encoding a peptide analog, whereby said first and second fragment are within the same open reading frame of said nucleic acid molecule;
- a nucleic acid sequence encoding an enzyme capable of dehydrating serine and/or threonine;
- optionally a nucleic acid sequence encoding a transporter protein; and
- a nucleic acid sequence encoding an enzyme capable of inducing lanthionine and/or methyllanthionine formation if the enzymatically formed intramolecular linkage has the general formula I as defined in claim 4;
b) allowing for the translation of said first nucleic acid;
c) harvesting said peptide analog; and
d) inducing lysinoalanine formation by exposing the peptide to a pH of between 8 and 11 if the enzymatically formed intramolecular linkage has the general formula II or III as defined in claim 5.

14. Method according to claim 13, further comprising selecting a cyclic peptide analog which has enhanced biological activity as compared to the linear counterpart GLP-1 or GLP-1 related peptide, preferably wherein said selecting comprises measuring said biological activity in an *in vitro* assay system using said cyclic peptide analog and a competitor, such as the linear counterpart GLP-1 or GLP-1 related peptide, preferably wherein said biological activity is extracellular cAMP production, intracellular cAMP production, or insulin production.

15. Method according to claim 13 or 14, wherein said second nucleic acid fragment encodes a peptide analog selected from table 1.
